# EUROPEAN PATENT SPECIFICATION

(11) **EP 1 830 833 B1**
(45) Date of publication and mention of the grant of the patent: **27.01.2010**
(21) Application number: 05849954.2
(22) Date of filing: 15.12.2005
(51) Int. Cl.: A61K 31/341, A61K 31/381, A61K 45/06, A61K 31/517, A61P 25/18

(54) **COMBINATION OF A GLYCINE TRANSPORTER (GLYT1) INHIBITOR AND AN ANTIPSYCHOTIC FOR THE TREATMENT OF SYMPTOMS OF SCHIZOPHRENIA AS WELL AS ITS PREPARATION AND USE THEREOF**
KOMBINATION AUS EINEM GLYCIN-TRANSPORTER (GLYT1) HEMMER UND EINEM ANTIPSYCHOTIKUM ZUR BEHANDLUNG VON SYMPTOMEN VON SCHIZOPHRENIE UND IHRE HERSTELLUNG UND VERWENDUNG
COMBINAISON D'UN INHIBITEUR DE TRANSPORTEUR GLYCINE (GLYT1) ET D'UN ANTIPSYCHOTIQUE DESTINEE AU TRAITEMENT DE SYMPTOMES DE LA SCHIZOPHRENIE AINSI QUE LA PREPARATION ET L'UTILISATION DE CE COMPOSE

(30) Priority: 16.12.2004 US 636575 P
(43) Date of publication of application: 12.09.2007
(73) Proprietor: Janssen Pharmaceutica NV, 2340 Beerse (BE)
(72) Inventor: MOYER, John, Allen, Buck County, PA 18901 (US); ASHTON, David, B-2300 Turnhout (BE); KALIVAS, Peter, Charleston, SC 29425 (US); KLITENICK, Mark, Bridgewater, NJ 08807 (US)
(74) Representative: Mercer, Christopher Paul
(86) International application number: PCT/US2005/045749
(87) International publication number: WO 2006/066121

(56) References cited:
- WO-A-2004/113301
- WO-A-2005/046601
- WO-A-2005/058317
- WO-A-2005/058885
- WO-A-2006/000222
- US-A- 5 837 730
- US-A1- 2002 169 197
- US-A1- 2003 092 769
- US-B1- 6 426 364
- TSAI GUOCHUAN ET AL: "Glycine transporter I inhibitor, N-methylglycine (sarcosine), added to antipsychotics for the treatment of schizophrenia." BIOLOGICAL PSYCHIATRY. 1 MAR 2004, vol. 55, no. 5, 1 March 2004 (2004-03-01), pages 452-456, XP002378149 ISSN: 0006-3223
- SUR C ET AL: "THE THERAPEUTIC POTENTIAL OF GLYCINE TRANSPORTER-1 INHIBITORS" EXPERT OPINION ON INVESTIGATIONAL DRUGS, ASHLEY PUBLICATIONS LTD., LONDON, GB, vol. 13, no. 5, 2004, pages 515-521, XP008052549 ISSN: 1354-3784

## Description

### CROSS REFERENCE TO RELATED APPLICATIONS

This application claims priority to Application No. 60/636,575 filed on December 16, 2004.

### FIELD OF THE INVENTION

The present invention relates to the prevention and treatment of symptoms of schizophrenia. More particularly, the invention relates to pharmaceutical compositions and treatments comprising an antipsychotic and a GlyT1 inhibitor for treating symptoms of schizophrenia associated with disorders such as schizophrenia, dementia; depression, Alzheimer's, ADHD, substance abuse and anxiety.

### BACKGROUND OF THE INVENTION

Traditional models of schizophrenia and bipolar disorder have focused on dopaminergic systems. Dopamine antagonists and/or lesions of dopamine pathways reduce the effects of phencyclidine (PCP) on two behavioral measures that are thought to have relevance to clinical symptomatology of schizophrenia: cognitive tasks involving work memory and locomoter activity. (Adams et al., 1998). Adams et al. discloses findings that indicate that activation of dopamine neurotransmission is not sufficient to sustain PCP induced locomotion and impairment of working memory and that glutamatergic hyperstimulation may account for psychotomimetic and cognitive-impairing effects of PCP. PCP induces a psychotic state that closely resembles schizophrenia by blocking neurotransmission mediated at N-methyl-D-aspartate (NMDA)-type glutamate receptors. (Javitt et al., 1997). PCP-like agents uniquely reproduce negative, cognitive and positive symptoms of schizophrenia. Positive symptoms are behavioral excesses generally considered psychotic (e.g., hallucinations, delusions, bizarre behavior), whereas negative symptoms denote a deficiency from normal behavior (e.g., a lack of normal social responsiveness, flat affect). Cognitive dysfunctions include impairment in working memory, executive functions, sustained attention, basic processing of sensory stimuli, verbal episodic memory and smooth pursuit eye movements. More recent models of schizophrenia now postulate that schizophrenia is associated with dysfunction or dysregulation of neurotransmission mediated at brain NMDA-type glutamate receptors. The NMDA model of schizophrenia raised the possibility that agents which augment NMDA receptor-mediated neurotransmission might be therapeutically beneficial in schizophrenia. The primary neurotransmitter acting at NMDA receptors is glutamate. However, NMDA receptor activity is also modulated by the amino acid glycine which binds to a selective modulatory site that is an integral component of the NMDA receptor complex. U.S. Patent No. 5,854,286 discloses the use of orally administered glycine, in dietary quantities, for the treatment of schizophrenia.

Glycine is considered a full agonist at the NMDA-associated glycine binding site (McBain et al., 1989). D-Serine, like glycine, is present in brain in high concentration and may serve as an endogenous ligand for the glycine binding site of the NMDA receptor complex (Schell et al., 1995). U.S. Patent No. 6,162,827 discloses the use of D-serine in the treatment of symptoms of psychosis.

Although the finding with glycine and D-serine support the use of full glycine-site agonists, others have proposed that partial agonists at the glycine site, such as the drug D-cycloserine, should be more effective than full agonists in the treatment of schizophrenia (see, e.g., U.S. Patent No. 5,187,171). Partial agonists bind to the same site as full agonists (i.e., glycine recognition site of the NMDA receptor complex), but potentiate channel opening to a smaller percent (typically 40-70% of the activation seen with full agonists, McBain et al., 1989). Clinical studies with D-cycloserine have provided support for the concept that partial glycine-site antagonists may be effective in the treatment of schizophrenia (reviewed in D'Souza et al., 1995), and, the degree of improvement seen in studies of D-cycloserine (reviewed in D'Souza et al., 1995) has been comparable in some circumstances to the degree of improvement observed following studies with glycine (reviewed in D'Souza et al., 1995) or D-serine (Tsai et al., 1998).

A second potential approach to augmentation of NMDA receptor-mediated neurotransmission is the administration of agents that inhibit glycine transporters in brain, thereby preventing glycine removal from active sites within the CNS. It has been known for many years that the brain contains active transport systems for glycine that may regulate brain levels (D'Souza, 1995). More recent studies demonstrated that glycine transporters are differentially expressed in different brain regions (Liu et al, 1993; Zafra et al., 1995) and may be co-localized with NMDA receptors (Smith et al., 1992). However, it has also been known for many years that extracellular glycine levels are beyond the level needed to saturate the NMDA-associated glycine binding site, making it unclear whether glycine transporters are, in fact, able to maintain subsaturating glycine levels in the immediate vicinity of NMDA receptors. This is a crucial issue in that, if glycine levels were already at or above saturating levels, additional glycine would not, on theoretical grounds, be able to stimulate NMDA functioning (Wood, 1995). As glycine is an obligatory co-agonist along with glutamate, at the NMDA receptor, it has been suggested that Gly-T1 functions to maintain subsaturation levels at the synaptic cleft. (Bergeron et al. 1998). If under physiological conditions, the glycine binding site of the NMDA receptor is indeed unsaturated, then modulation of synaptic glycine concentrations using a Gly-T1 inhibitor would be a method of potentiating NMDA receptor function. (Slassi et al. 2004). This approach is attractive because it could avoid the toxic effects of agonists that directly act on the NMDA receptor.

Schizophrenia is a cognitive and behavioral disorder that affects up to 1% of the human population. Other disease states exhibit symptoms also seen in schizophrenia. Current understanding of the etiology of the symptoms of schizophrenia and similar disease states remains vague, but points to a combination of genetic and environmental factors. The search for medications to treat schizophrenia and similar disease states has traditionally focused on dopamine receptor antagonists, and more recently on drugs that combine dopamine receptor blockade with antagonist/agonist actions at other receptors. Based upon work with animal models, and the fact that blockade of NMDA glutamate receptors in normal humans produces schizophrenia-like symptoms, it has been postulated that hypofunction of the glutamate system, specifically at the NMDA receptor, underlies some symptoms in schizophrenia (Goff and Coyle, 2001). However, drugs that directly inhibit or stimulate NMDA receptors have proven unsafe for routine patient care in animal models and early clinical trials. Fortunately, the NMDA receptor is a complex heteromeric channel that can be pharmacologically modulated in more subtle ways than simply blocking or stimulating the glutamate binding site (Nakanishi et al., 1998).

Glycine is an obligatory co-agonist at the NR1 subunit of the NMDA type glutamate receptor complex. Thus, increasing tone on the glycine binding site (i.e., increasing extracellular glycine) potentiates the capacity of glutamate to open the NMDA channel. Given the proposed reduction in NMDA activity in schizophrenia, it has been postulated that elevating extracellular glycine may increase NMDA conductances and thereby relieve some symptoms of schizophrenia and similar disease states. Parsons et al. (1998) and Danysz et al. (1998) provide reviews of data related to the role of the NMDA receptor in a wide range of CNS disorders.

U.S. Patent No. 6,355,681 discloses the use of glycine and precursors in the treatment of symptoms of psychosis.

U.S. Application No. 20020161048 discloses the use of glycine substitutes and precursors in the treatment of symptoms of psychosis.

U.S. Application No. 20020183390 discloses a method and composition for augmenting NMDA receptor mediated transmission involving the use of a D-serine transport inhibitor. U.S. Application No. 20020183390 discloses that the method and composition may be used in the treatment of neuropsychiatric disorders such as schizophrenia.

One mechanism for increasing extracellular glycine is to prevent its elimination from the extracellular space by uptake through the glycine transporter 1 (GlyT1) (Javitt and Frusciante,1997). Aragon et al. (2003) is a review on the localization, transport mechanism, structure, regulation and pharmacology of glycine transport inhibitors. Javitt (2002) discloses that NMDA receptor dysfunction may play a role in the pathophysiology of schizophrenia. Javitt (2002) also discloses that GlyT1 inhibitors may represent a "next generation" approach to the treatment of the persistent negative and cognitive symptoms of schizophrenia.

U.S. Patent No. 5,837,730 discloses that a glycine transport inhibitor, glycyldodecylamide (GDA), is able to exert glycine-like behavioral effects in rodents.

None of the references cited disclose or suggest the GlyT1 inhibitor and antipsychotic combination of the invention.

### SUMMARY OF THE INVENTION

An object of the present invention is a pharmaceutical composition comprising an antipsychotic and a GlyT1 inhibitor according to claim 1.

Disclosed herein for information purposes only is a method for treating symptoms of schizophrenia which comprises administration of a combination of an antipsychotic and a GlyT1 inhibitor.

Disclosed herein for information purposes only is a method for increasing extracellular glycine levels in a mammal, which comprises administration of an antipsychotic in combination with a GlyT1 inhibitor.

Disclosed herein for information purposes only is a method for increasing extracellular dopamine levels in a mammal which comprises administration of an antipsychotic in combination with a GlyT1 inhibitor.

### BRIEF DESCRIPTION OF THE FIGURES

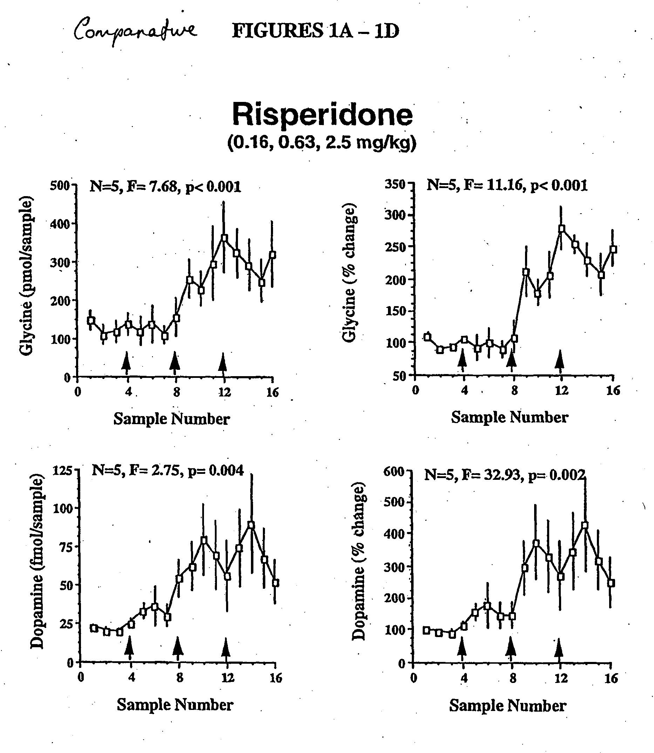

Comparative Figures 1A-1D: Experiment #1, Effects of risperidone on extracellular glycine (upper panels) and dopamine (lower panels) in the rat striatum. Doses were given in ascending order as indicated by the arrows. Data in the right panels were normalized to the percent change from the average of the three baseline values (i.e., the data obtained before the first arrow). Data are shown as mean ± sem, and were statistically evaluated using a one-way ANOVA with repeated measures over time.

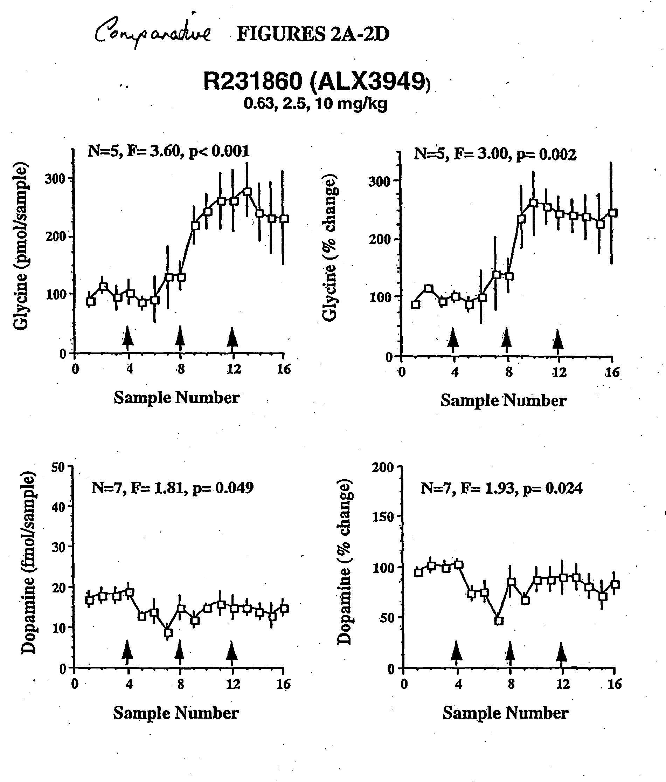

Comparative Figures 2A-2D: Experiment #2, Effects of COMPOUND NO. 1 on extracellular glycine (upper panels) and dopamine (lower panels) in the rat striatum. Doses were given in ascending order as indicated by the arrows. Data in the right panels were normalized to the percent change from the average of the three baseline values (i.e., the data obtained before the first arrow). Data are shown as mean ± sem, and were statistically evaluated using a one-way ANOVA with repeated measures over time.

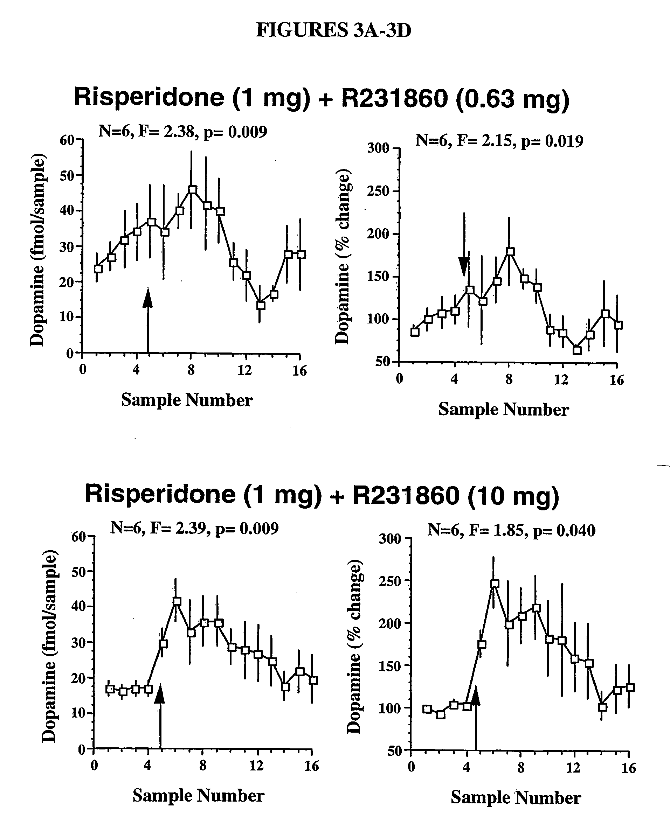

Figures 3A-3D: Experiment 3: Effects of a combination of risperidone and COMPOUND NO. 1 on extracellular glycine in the striatum. Drug co-administration was made at the arrow. Data in the right panels were normalized to the percent change from the average of the three baseline values (i.e., the data obtained before the first arrow). Data are shown as mean ± sem, and were statistically evaluated using a one-way ANOVA with repeated measures over time. Effects of a combination of risperidone and COMPOUND NO. 1 on Extracellular dopamine in the striatum. Drug co-administration was made at the arrow. Data in the right panels were normalized to the percent change from the average of the three baseline values (i.e., the data obtained before the first arrow). Data are shown as mean ± sem, and were statistically evaluated using a one-way ANOVA with repeated measures over time.

### DETAILED DESCRIPTION OF THE PREFERRED EMBODIMENTS

It has now been found that combining an antipsychotic with a GlyT1 inhibitor in the treatment of symptoms of schizophrenia results in an unexpected increase in extracellular glycine.

The present invention is directed to an antipsychotic/GlyT1 inhibitor combination according to claim 1. The antipsychotic and the GlyT1 inhibitor of the combination may each be administered separately or may be together in a single pharmaceutical composition. The antipsychotic/GlyT1 inhibitor combination may be used in the treatment of symptoms of schizophrenia associated with disorders such as schizophrenia, dementia, depression, Alzheimer's, ADHD, substance abuse and anxiety.

A number of compounds, including COMPOUND NO.1 and Compound No. 2, bind to and inhibit glycine uptake through GlyT1. GlyT1 inhibitors that may be used in accordance with the invention therefore include:
Compound No. 1, which is disclosed in U.S. Patents Nos. 6,426,364; 6,525,085; and 6,579,987. 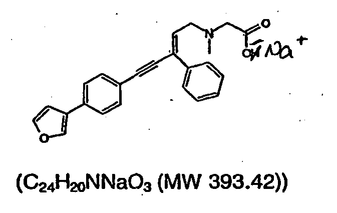
Compound No. 2, which is disclosed in U.S. Patents Nos. 6,426,364; 6,525,085; and 6,579,987.

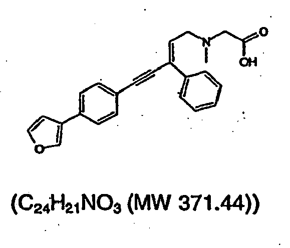

Antipsychotics may be used in accordance with the invention are atypical antipsychotics. Atypical antipsychotics include Risperidone, 3-[2-[4-(6-fluoro-1,2-benzisoxazol-3-yl)piperidino]ethyl]-2-methyl-6,7,8,9 -tetrahydro-4H-pyrido-[1,2-a]pyrimndin-4-one, and its use in the treatment of psychotic diseases are described in U.S. Pat. No. 4,804,663.

Also included are pharmaceutically acceptable salts thereof, pharmaceutically acceptable esters thereof, and enantiomeric forms of the atypical antipsychotics.

### EXAMPLES

### SUMMARY OF RESULTS

Dopamine transmission microdialysis studies were conducted to determine if COMPOUND NO. 1 affected dopamine transmission in the brain. Drugs inhibiting dopamine transmission are to date the most effective medications against schizophrenia. Likewise, it is known that most drugs effective in schizophrenia antagonize D2 dopamine autoreceptors and thereby elevate extracellular dopamine (Ferre et al., 1995). To determine if COMPOUND NO. 1 was synergistic with this action, COMPOUND NO. 1 was combined with the antipsychotic risperidone and effects on both glycine and dopamine were quantified in the striatum.

A cumulative dose-response curve for COMPOUND NO. 1 revealed the expected dose-dependent increase in extracellular glycine levels in the striatum. While the lowest dose (0.63 mg/kg) was without effect, the highest dose of COMPOUND NO. 1 (10 mg/kg) caused a 2.5-fold increase in glycine. Although without affect on glycine, the lowest dose of COMPOUND NO. 1 produced a significant reduction in extracellular dopamine, and following administration of 2.5 mg/kg the levels of dopamine were normalized and remained unaltered even by the highest dose of COMPOUND NO. 1.

Risperidone produced a dose-dependent elevation in both extracellular dopamine and glycine. While the effect on dopamine was expected due to blockade of D2 autoreceptors, the marked rise in glycine was unexpected. Similar to dopamine, the elevation in glycine occurred at a threshold dose of 0.16 mg/kg risperidone. Indeed risperidone was equally effective at producing a rise in extracellular glycine as COMPOUND NO. 1, as indicated by a 2.5-fold increase in glycine after 2.5 mg/kg risperidone.

The effects of each drug alone were additive when the drugs were given together. That is to say, the combination of COMPOUND NO. 1 and risperidone produced a greater increase in glycine than either drug alone, reaching a 6-fold increase following a combination of 1 mg/kg risperidone and 10 mg/kg COMPOUND NO. 1. Also, commensurate with the biphasic reduction in dopamine by COMPOUND NO. 1, it was found that the combination of low dose COMPOUND NO. 1 (0.63 mg/kg) tended to inhibit the increase produced by 1 mg/kg risperidone, while the higher dose of COMPOUND NO. 1 did not alter risperidone-induced elevations in dopamine.

### EXPERIMENTAL PROCEDURES

Compounds (as a Na salt) were stored, dissolved, and administered according to detailed instructions accompanying each compound. Compounds were dissolved in a solvent consisting of 10% BCD (beta cyclodextrin) and administered subcutaneously.

Male Sprague Dawley rats weighing 275-325 g at the start of the experiment were individually housed in a temperature-controlled colony room with a 12-h light/dark cycle. Food and water was available ad libitum throughout the experiment. The housing conditions and care of the animals were in accordance with the "Guide for the Care and Use of Laboratory Animals" (Institute of Laboratory Animal Resources on Life Sciences, National Research Council, 1996).

Guide cannula were stored in 95% ETOH prior to surgery, whereas surgical tools underwent heat sterilization (250°C) immediately before each surgery. Rats were anesthetized using a ketamine hydrochloride (100 mg/kg, IP) xylazine (12 mg/kg) mixture. After adequate anesthesia had been determined (using toe and tail pinch procedures), rats were placed into a stereotaxic instrument. The skull region was wiped with a 2% Betadine solution and a rostrocaudal incision was made to expose the surface of the skull. Bilateral guide cannula (20 gauge; Plastics One) were chronically implanted over the medial striatum (A/P: +0.5, M/L: ±2.5, D/V: -2.0; Paxinos & Watson, 1998) and secured using four skull screws and cranioplastic cement. The cannula need to be implanted at an angle to obtain the minimum inter-cannula distance needed for our probe leash used during microdialysis sampling. Following surgery, body temperature was maintained using a heating pad and the rats were monitored until fully conscious. Rats were then individually housed and assessed daily by monitoring general activity, body weight, and feces. Rats were monitored for signs of an infection and cefazolin (100 mg/kg; intramuscular) was available as needed. Notation was made of any animal administered antibiotic.

Rats were given at least five days to recover prior to microdialysis sampling. Approximately 18 hr prior to sampling, a microdialysis probe (24 gauge; 2-3 mm exposed membrane; 13000 MWCO) encased in a spring leash and attached to a liquid swivel connected to a balancing arm was inserted into the guide cannula of an awake rat. The probe was secured in place by screwing a threaded portion of the probe leash onto the guide cannula. The rat was then placed into a behavioral chamber (Omnitech, Columbus OH) equipped with a fan and house light (10W), and food and water was available ad libitum. On the day of the experiment, dialysis buffer consisting of 5 mM glucose, 140 mM NaCl, 1.4 mM CaCl₂, 1.2 mM MgCl₂, and 0.15% phosphate buffer saline, pH 7.4, was perfused through the probe (2.0 µl/min) at least two hr prior to sample collection. Twenty-min dialysis samples were then collected for two hr to determine basal glycine levels. Rats were then injected (intraperitoneal) with vehicle or one dose of the test compound, and 30-min samples were collected for up to 10 hr. Samples were split for separate chromatographic evaluation of glycine and dopamine, and frozen (-80°C) until analyzed.

Rats, with the dialysis probes in place, were given an overdose of pentobarbital, and the brains fixed by intracardiac infusion of PBS-formalin. Coronal brain sections were 100 µM thick and stained with cresyl violet to verify probe placements. Probes were left in place as the animal was perfused in order to check for the presence of blood in the probe tract.

The concentrations of glycine and dopamine in dialysate samples was determined using a Waters Alliance 2690 HPLC system with fluorometeric detection or an ESA coulometric electrochemical detection HPLC system, respectively. Dialyis samples were split between the two systems enabling both dopamine and glycine to be measured in each sample. A Waters Spherisorb ODS2 column (5 µM, 4.6 x 250 mm) was used to separate the amino acids. Glycine was detected using a Waters 474 Fluorescence Detector with an excitation wavelength of 320 nm and an emission wavelength of 400 nm. The mobile phase consisted of 80% H₂O, 20% acetonitrile, 0.1 M Na₂HPO₄, and 0.1 mM ethylenediamine-tetraacetic acid (pH to 5.8 with phosphoric acid; 0.2 µm filter) with a flow rate of 0.75 ml/min. Samples were placed into the refrigerated autosampler (4°C) and precolumn derivatization of the amino acids with o-phthaldehyde was performed using the Waters Alliance System. A total of 15 µl (5 µl sample plus 15 µl OPA) was injected onto the column. All samples collected 2 hr before and after treatment were analyzed. For the samples collected during post-treatment hrs 2-24, one 20-min sample/hr was analyzed; the other two samples were retained for analysis of compound within the sample. Glycine peak heights were compared to an external standard curve for quantification. A new standard curve was generated each day.

For dopamine analysis, samples were placed in an ESA (Chelmsford, MA) Model 540 autosampler connected to an HPLC system with electrochemical detection. Separation was achieved by pumping the samples through a 15 cm C₁₈ reversed phase column (ESA, Inc.) and then samples were reduced/oxidized using coulometric detection. Three electrodes were used: a guard cell (+400 mV), a reduction analytical electrode (-150 mV) and an oxidation analytical electrode (+250 mv). Peaks were recorded and the area under the curve measured by a computer running ESA Chromatography Data System. These values were normalized by comparison to an internal standard curve for isoproteronol and quantified by comparison to an external standard curve.

Peak heights were compared to an external standard curve for quantification. The data was normalized to percent change from baseline (mean of 3 30-min samples prior to treatment). In addition, raw data was furnished and differences were reported along with the normalized data. All data was evaluated using a one-way ANOVA with repeated measures over time using the Statview program on a G4 MacIntosh.

Comparative Experiment #1. Extracellular levels of dopamine and glycine in the striatum of the rat (N=5) were assessed following administration of risperidone at three (3) doses (ascending; 0.16, 0.63 and 2.5 mg/kg). Dialysis samples were obtained every 30 min, each dose being assessed for 2 hours (8 hours total). Comparative FIGURES 1A-1D show the effect of risperidone on glycine and dopamine. The data are shown both as amount of analyte per sample, as well as normalized to the percent change from the average of the baseline values (i.e., samples obtained before the first drug injection). Risperdone produced the expected elevation in extracellular dopamine, with the lowest dose eliciting a threshold elevation of approximately 50%, and the two higher doses producing a 3-4 fold increase in dopamine. Surprisingly, a similar elevation in extracellular glycine was observed following risperidone. Although the lowest dose was without effect, the two higher doses of risperidone elicited a dose-dependent elevation in glycine up to a maximum 2.5-fold increase.

Comparative Experiment #2. Extracellular levels of dopamine and glycine in the striatum of the rat (N=5) were assessed following administration of COMPOUND NO. 1 at three (3) doses (ascending; 0.63, 2.5 and 10 mg/kg). Dialysis samples were obtained every 30 min, each dose being assessed for 2 hours (8 hours total). Comparative FIGS 2A-2D show the results of this experiment. As expected, COMPOUND NO. 1 elicited a dose-dependent elevation in extracellular glycine. A threshold effect was seen after 0.63 mg/kg, and 10 mg/kg produced a 2.5-fold elevation in glycine. The effect of COMPOUND NO. 1 on extracellular dopamine was biphasic with respect to dose (N=7). The lowest dose of COMPOUND NO. 1 produced a nearly 50% reduction in extracellular dopamine. The levels of dopamine returned to normal following injection an injection of 2.5 mg/kg and remained unaltered by the highest dose of COMPOUND NO. 1.

Experiment #3. The data generated from Experiments #1 and #2 was assessed to determine the best combination of doses of COMPOUND NO. 1 and risperidone in order to determine synergism or antagonism between the two compounds. Two dosing regimens were identified. In order to evaluate the effect of the low dose of COMPOUND NO. 1 on dopamine (see Comparative FIGURES 2A-2D), a combination of 0.63 mg/kg COMPOUND NO. 1 and 1.0 mg/kg risperidone was administered in a single bolus injection. In order to examine for a potential synergism between COMPOUND NO. 1 and risperidone in elevating extracellular glycine (see Comparative FIGURES 1A-1D and 2A-2D), 10 mg/kg COMPOUND NO. 1 and 1.0 mg/kg risperidone was administered in a single bolus injection.

Effects on glycine: The lower dose of COMPOUND NO. 1 (0.63 mg/kg) and the dose of risperidone examined each produce a modest rise in glycine when given alone (see Comparative FIGURES 1A-1D and 2A-2D, respectively). When the two doses of drug were co-administered (N=5) there was an approximate doubling of extracellular glycine that was consistent with the effect of risperidone alone. However, combining risperidone with the higher dose of COMPOUND NO. 1 (10 mg/kg) caused a clear additive effect (N=6). Thus, while each drug alone produced a 2-3 fold elevation in glycine, combined there was a 6-fold increase in extracellular glycine.

Effects on dopamine: FIGURES 3A-3D illustrate the effect of both drug combinations on extracellular dopamine in the striatum. The upper panels illustrate the effect of combining the lower dose of COMPOUND NO. 1 (0.63 mg/kg) with risperidone (N=6). This dose of COMPOUND No. 1 reduced extracellular dopamine, and it can be seen that, although a significant increase was measured, COMPOUND NO. 1 partly antagonized the increase in dopamine expected following 1.0 mg/kg risperidone. Thus, the expected 300% increase in dopamine following this dose of risperidone (see Comparative FIGURES 1C-1D) was reduced to 150% when given in combination with COMPOUND NO. 1 (0.63 mg/kg). In contrast, the higher dose of COMPOUND NO. 1 (10 mg/kg) alone was without effect on dopamine (Comparative FIGURES 2C-2D), and when co-administered did not alter the capacity of risperidone to elevate extracellular dopamine (N=6).

### Discussion and Conclusions

These data reaffirm the capacity of the GlyT1 antagonist COMPOUND NO. 1 to produce a dose-dependent elevation in extracellular glycine, and demonstrate that at lower doses, this drug reduces extracellular dopamine. The present data also affirm the findings of others that risperidone elevates extracellular dopamine, and makes the surprising and important observation that risperidone produces a dose-dependent elevation in extracellular glycine. Moreover, in combination, the two drugs appear additive in their effects on glycine, indicating separate mechanisms of action.

COMPOUND NO. 1 and dopamine. It was surprising that COMPOUND NO. 1 reduced extracellular dopamine. The fact that this was observed only at lower doses may indicate a separate mechanism of action than GlyT1 blockade. Regardless of the mechanism, this effect is synergistic with known therapeutic actions of antipsychotic medications. Thus, reducing dopamine transmission in the striatum may be indicative of a mechanism for reducing dopamine receptor tone that is distinct from the classic D2 receptor blockade associated with most antipsychotic drugs. While this effect in the striatum (especially ventral striatum) is thought to be an important therapeutic action of antipsychotic drugs, reducing dopamine transmission in the prefrontal cortex would be expected to exacerbate the cognitive impairment associated with schizophrenia. However, there are known instances where pharmacological and environmental challenges differentially affect prefrontal cortical and striatal dopamine transmission, notably in relation to NMDA receptor blockade (Cabib and Puglisi-Allegga, 1996; Moghaddam and Adams, 1998), and the effects of COMPOUND NO. 1 on extracellular dopamine in striatum may not predict effects in the prefrontal cortex. This would be especially true if the effects on dopamine were indirect since the synaptic organization of the prefrontal cortex differs markedly from the striatum.

The slight antagonism by low dose COMPOUND NO. 1 (0.63 mg/kg) of the effect of risperidone to elevate dopamine in the striatum is potentially important, especially if the effect of COMPOUND NO. 1 is distinct in the cortex and striatum. Thus, antagonism of risperidone in the striatum, but not in the cortex could have therapeutically beneficial impact, given that risperidone actions in the striatum are thought to mediate untoward motor side-effects.

The elevation in glycine by risperidone was unexpected. The mechanism remains unclear. Given that elimination of glycine from the extracellular space is primarily by glycine uptake, antagonism of the transporter is one option. While it is unlikely that risperidone binds directly to GlyT (Goff and Coyle, 2001), it is possible that blockade of dopamine (or serotonin) receptors may regulate GlyT.

Regardless of the mechanism by which risperidone elevates glycine, there is a clear additive effect between COMPOUND NO. 1 and risperidone with regard to elevating extracellular glycine. Inasmuch as schizophrenia may in part result from reduced NMDA conductance, the additive effect on extracellular glycine may provide therapeutic benefit by indirectly potentiating NMDA conductances. Thus if in fact elevating glycine is of therapeutic benefit, combining COMPOUND NO. 1 with risperidone may permit the use of lower doses of risperidone.

This study identified two novel actions of COMPOUND NO. 1 and risperidone. Low doses of COMPOUND NO. 1 reduced dopamine levels and risperidone produced a dose-dependent elevation in glycine. While the cellular mechanisms mediating these actions remain unclear, they result in potentially important interactions between the two drugs. Thus, COMPOUND NO. 1 (0.63 mg/kg) slightly antagonized the capacity of risperidone to elevate dopamine, while the capacity of both drugs to elevate glycine was additive. Inasmuch as dopamine and glutamate are involved in the etiology or symptomatology of schizophrenia the interactions of COMPOUND NO. 1 with the known antipsychotic risperidone is therapeutically relevant.

### References

Adams et al. 1998. Corticolimbic dopamine neurotransmission is temporarily dissociated from the cognitive and locomotor effects of phencyclidine. J. Neuroscience, 18(14):5545-5554.
Aragon et al. 2003. Structure, function and regulation of glycine neurotransmitters. European Journal of Pharmacology, 479:249-262.
Bergeron et al. 1998. Modulation of N-methyl-D-aspartate receptor function by glycine transport. Proc. Natl. Acad. Sci. U.S.A., 95(26):15730-15735.
Cabib S, Publisi-Allegra S. 1996. Stress, depression and the mesolimbic dopamine system. Psychopharmacology, 128: 331-342.
Danysz et al. 1998. Glycine and N-methyl-D-aspartate receptors: physiological significance and possible therapeutic applications.
D'Souza et al. 1995. Glycine site agonists of the NMDA receptor: a review. CNS Drug Revs., 1:227-260.
Ferre S, et al. 1995. Antagonistic interaction between A2a receptors and D2 receptors: implications for the treatment of schizophrenia. Neuroscience, 63: 765-773.
Goff DC, Coyle JT. 2001. The emerging role of glutamate in the pathophysiology and treatment of schizophrenia. Am J Psychiatry, 158: 1367-1377.
Javitt DC, Frusciante M. 1997. Glycyldodecylamide, a phencyclidine behavioral antagonist, blocked cortical glycine uptake: implications for schizophrenia and substance abuse. Psychopharmacology, 129: 96-98.
Javitt DC. 2002. Glycine modulators in schizophrenia. Current Opinion in Investigational Drugs, 3(7):1067-1072.
McBain et al. 1989. Structural requirements for glycine co-agonist site of N-methyl-D-aspartate receptors expressed in Xenopus oocytes. Mol. Pharmacol., 36:556-65.
Liu et al. 1993. Cloning and expression of a spinal cord - and brain - specific glycine transporter with novel structural features. J. Biol. Chem., 268: 22802-22808.
Moghaddam B, Adams B. 1998. Corticolimbic dopamine transmission is temporally dissociated from the cognitive and locomotor effects of phencyclidine. J. Neuroscience, 18: 5545-5555.
Newcomer et al. 2001. NMDA receptor regulation of memory and behavior in humans. Hippocampus, 11:529-542 (2001).
Nakanishi Y, et al. 2001. Glutamate receptors: brain function and signal transduction. Brain Res Rev, 26: 230-235.
   Parsons et al. 1998. Glutamate in CNS disorders as a target for drug development: an update. Drug News Perspect., 11(9):523-579.
Schell MJ, et al. 1995. D-serine, an endogenous synaptic modulator: localization to astrocytes and glutamate-stimulated release. Proc. Natl. Acad. Sci. USA, 92:3948-3952.
Slassi et al. 2004. Recent progress in the use of glycine transporter-1 inhibitors for the treatment of central and peripheral nervous system diseases, Ashley Publications Ltd ISSN 1354-3776:201-214.
Smith et al. 1992. Cloning and expression of a glycine transporter reveal colocalization with NMDA receptors. Neuron, 8: 927-935.
Tsai et al. 1998. D-serine in the treatment of schizophrenia. Biol. Psychiatry, 44:1081-1089.
Zafra et al. 1995. Glycine transporters are differentially expressed among CNS cells. J. Neuroscience, 15: 3952-3969.

## Claims

1. A medical product, comprising
(a) an antipsychotic selected from the group consisting of risperidone, paliperidone, and pharmaceutically acceptable salts thereof, pharmaceutically acceptable esters thereof and enantiomeric forms thereof, and
(b) a GIyT1 inhibitor selected from the group consisting of 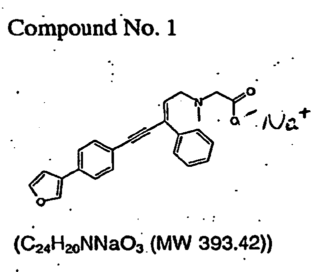 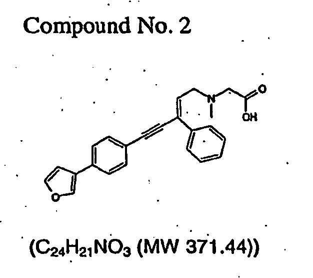 and pharmaceutically acceptable salts thereof, pharmaceutically acceptable esters thereof and enantiomeric forms thereof;
as a combined preparation for simultaneous, separate or sequential use in treating or preventing symptoms of schizophrenia associated with disorders such as schizophrenia, dementia, depression, Alzheimer's ADHD, substance abuse and anxiety.

2. The medical product of claim 1 administered in an amount effective to treat or prevent said symptoms.

3. The medical product of claim 1 administered to a mammal to increase extracellular glycine in a mammal.

4. The medical product of claim 3, wherein said extracellular glycine is increased to an amount more than if said antipsychotic or said GIyT1 inhibitor was administered alone.

5. The medical product of claim 3, wherein said GIyT1 inhibitor has minimal or no effect on extracellular dopamine.

## Patentansprüche

1. Medizinisches Produkt, umfassend
(a) ein Antipsychotikum, das ausgewählt ist aus der Gruppe, bestehend aus Risperidon, Paliperidon und pharmazeutisch verträglichen Salzen davon, pharmazeutisch verträglichen Estern davon und enantiomeren Formen davon, und
(b) einen GlyT1-Hemmer, der ausgewählt ist aus der Gruppe, bestehend aus 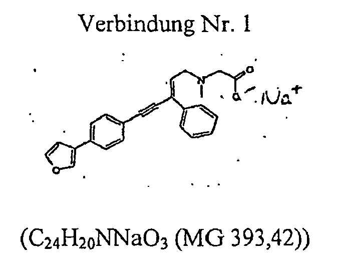 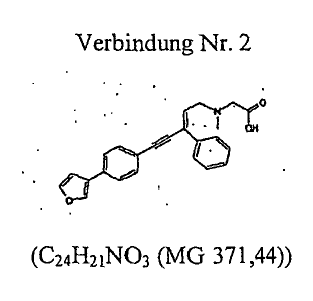 und pharmazeutisch verträglichen Salzen davon, pharmazeutisch verträglichen Estern davon und enantiomeren Formen davon;
als eine kombinierte Zubereitung für gleichzeitige, getrennte oder sequentielle Verwendung zur Behandlung oder Verhinderung von Symptomen von Schizophrenie im Zusammenhang mit solchen Störungen wie Schizophrenie, Demenz, Depression, Alzheimer-Krankheit, ADHD, Substanzmissbrauch und Angst.

2. Medizinisches Produkt nach Anspruch 1, das in einer Menge verabreicht wird, die darin wirksam, die Symptome zu behandeln oder zu verhindern.

3. Medizinisches Produkt nach Anspruch 1, das einem Säuger verabreicht wird, um extrazelluläres Glycin in einem Säuger zu erhöhen.

4. Medizinisches Produkt nach Anspruch 3, wobei das extrazelluläre Glycin bis zu einer Menge erhöht wird, die größer ist, als wenn das Antipsychotikum oder der GlyT1-Hemmer allein verabreicht würde.

5. Medizinisches Produkt nach Anspruch 3, wobei der GlyT1-Hemmer minimalen oder keinen Effekt auf extrazelluläres Dopamin hat.

## Revendications

1. Produit médical, comprenant
(a) un antipsychotique sélectionné dans le groupe consistant en rispéridone, palipéridone, et des sels pharmaceutiquement acceptables de celui-ci, des esters pharmaceutiquement acceptables de celui-ci et des formes énantiomériques de celui-ci, et
(b) un inhibiteur du GlyT1 sélectionné dans le groupe consistant en 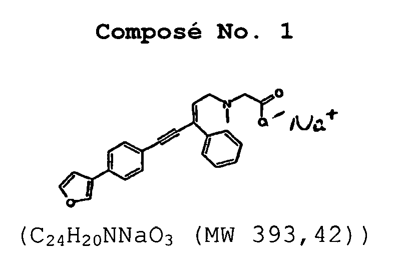 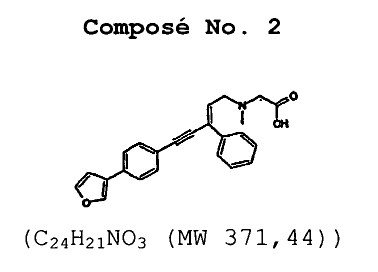 et des sels pharmaceutiquement acceptables de ceux-ci, d'esters pharmaceutiquement acceptables de ceux-ci et de formes énantiomériques de ceux-ci;
comme une préparation combinée pour l'utilisation simultanée, séparée ou séquentielle pour le traitement ou la prévention de symptômes de la schizophrénie associée à des troubles comme une schizophrénie, démence, dépression, ADHD d'Alzheimer, abus de substance et anxiété.

2. Produit médical selon la revendication 1, administré en une quantité efficace pour traiter ou prévenir lesdits symptômes.

3. Produit médical selon la revendication 1, administré à un mammifère pour augmenter la glycine extracellulaire dans un mammifère.

4. Produit médical selon la revendication 3, où ladite glycine extracellulaire est augmentée à une quantité supérieure que si ledit antipsychotique ou ledit inhibiteur du GlyT1 était administré seul.

5. Produit médical selon la revendication 3, où ledit inhibiteur de GlyT1 a un effet minimal ou n'a pas d'effet sur la dopamine extracellulaire.
